# EUROPEAN PATENT SPECIFICATION

(11) **EP 1 305 057 B1**
(45) Date of publication and mention of the grant of the patent: **22.06.2005**
(21) Application number: 01959403.5
(22) Date of filing: 31.07.2001
(51) Int. Cl.: A61L 27/20, A61L 27/50, A61L 31/04, A61L 31/14

(54) **MICROPARTICULATE BIOMATERIAL COMPOSITION OF HYALURONIC ACID FOR MEDICAL USE**
BIOMATERIAL IN FORM VON MIKROPARTIKELN VON HYALURONSAURE ZUR MEDIZINISCHEN VERWENDUNG
COMPOSITION DE BIOMATIERE MICROPARTICULAIRE D'ACIDE HYALURONIQUE A USAGE MEDICAL

(30) Priority: 31.07.2000 US 629000
(43) Date of publication of application: 02.05.2003
(73) Proprietor: Iscience Corporation, San Francisco, CA 94117 (US)
(72) Inventor: YAMAMOTO, Ronald, K., San Francisco, CA 94089 (US); CONSTON, Stanley, R., San Carlos, CA 94070 (US)
(74) Representative: Hubert, Philippe
(86) International application number: PCT/US2001/024149
(87) International publication number: WO 2002/009787

(56) References cited:
- WO-A-00/37124
- WO-A-99/11196
- FR-A- 2 733 426
- FR-A- 2 759 576
- US-A- 5 752 974
- CRANDALL A S: "NONPENETRATING FILTERING PROCEDURES: VISCOCANALOSTOMY AND COLLAGEN WICK" SEMINARS IN OPHTHALMOLOGY, SAUNDERS, US, vol. 14, no. 3, September 1999 (1999-09), pages 189-195, XP001004444 ISSN: 0882-0538

## Description

### Field of Invention:

The present invention is directed to an injectable biomaterial for the localized treatment of tissues.

### Background of Invention:

A variety of biocompatible materials, or biomaterials, have been used as medical implants to act as a surgical aid in maintaining a tissue space, to appose tissue or to increase the bulk of tissue in a localized area. Early examples include the use of silicone rubber materials used for permanent soft tissue reconstruction of the chin and nose. Later development of biodegradable biomaterials allowed the use of materials such as reconstituted bovine collagen and hydrolytically degradable synthetic polymers such as polylactic acid, polyglycolic acid, and their copolymers. Such degradable biomaterials can allow the body to slowly absorb the implant while replacing the space with new tissue. Examples of such biomaterial applications include porous collagen implants used as synthetic skin and polylactic acid implants used for bone fixation.

Biomaterials which are injected and degradable have particular advantage in surgery due to the ability to access tissues areas with minimally invasive surgical tools. An example is the use of collagen fibril dispersions (Zyderm, Collagen Corporation) injected into the tissues around the urethral sphincter in the treatment of incontinence and also for the augmentation of soft tissues for cosmetic purposes. Similar injectable materials have been described from a variety of compositions, including liquid copolymers in US Patent 5,824,333 and dextran microparticles in US Patent 5,633,001.

Most prior art surgical applications of biomaterials result in the formation of fibrotic tissue and subsequent tissue ingrowth into the region previously occupied by the biomaterial. In certain surgical applications, it is desired to locally apply a material to tissues to maintain space but also prevent tissue ingrowth into the area. For example, in the surgical repair of nerves, eyes, and abdominal organs, the resulting fibrosis may complicate or negate the effect of the surgical repair. It is desirable in the case of these types of surgical procedures to have an injectable biomaterial, which can be applied to tissues to form an implant, but prevents tissue ingrowth or proliferation of fibroblasts and fibrous tissues.

A particular application is with a recently developed surgical treatment for the eye known as viscocanalostomy. The procedure involves surgically opening a flap of the sclera and dissecting down to de-roof Schlemm's Canal to increase aqueous drainage. A high viscosity solution, known as a viscoelastic, is injected into the canal to dilate it, and may act to open the trabecullar meshwork from the canicular space to increase flow of the aqueous and reduce intraocular pressure. The viscoelastic also acts as a fibrosis inhibitor, reducing the influx of fibroblastic cells from the healing response, which would negate the effects of the procedure by blocking fluid flow.

The predominant viscoelastic material used in ophthalmic procedures is a high viscosity liquid comprised of high molecular weight hyaluronic acid (HA) or sodium hyaluronate, which is a glycosoaminoglycan component found in several human tissues including the eye and synovial fluid of the joints. Due to the extremely high viscosity of high molecular weight HA solutions, the formulations used in these procedures are on the order of 0.5-1% HA in solution. HA and its derivatives have been used in ophthalmic applications for many years as solutions for phacoemulsfication of the eye during cataract removal. While suitable for the dilation of Schlemm's Canal and other tissues, current viscoelastic materials do not have the residence time in-vivo and fluid transport characteristics to provide a long-term maintenance of the surgical repair. It is desirable, in the instance of surgically treating tissue spaces such as Schlemm's Canal, to have an injectable material with bulking properties to effect dilation and maintain the surgical space for fluid flow, a long term degradation profile, and inhibition of the fibrosis associated with wound healing.

The present invention as defined in the claims describes biocompatible, injectable microsphere compositions and formulations which may be applied to tissues for such purposes.

### Known prior art:

US Patent 5,985,354 Nov 16, 1999 Mathiowitz, et al.
Preparation of multiwall polymeric microcapsules from hydrophilic polymers

US Patent 5,922,357 Jul 13, 1999 Coombes, et al.
Polymer microspheres and a method of production thereof

WO 99/11196 Mar 11, 1999 Conston, et. al.
Injectable tissue reconstruction material

EP 0265116 Nov 3, 1998 Della Valle, et al.
Cross-linked ester of hyaluronic acid

US Patent 5,824,333 Oct 20, 1998 Scopelianos, et al.
Injectable liquid copolymers for soft tissue repair and augmentation

US Patent 5,633,001 May 27, 1997 Ågerup
Composition and a method for tissue augmentation

US Patent 5,143,724 Sept 1, 1992 Leshchiner, et al.
Biocompatible viscoelastic gel slurries, their preparation and use

WO 90/09401 Aug 23, 1990 Malson et al.
Crosslinked hyaluronate gels, their use and method for producing them

US Patent 4,582,640 Apr 15, 1986 Smestad, et al.
Injectable cross-linked collagen implant material

WO 86/00079 Jan 3, 1986 Malson, et al.
Gel of crosslinked hyaluronic acid for use as a vitreous humor substitute

Obstbaum, S., M.D. et al., Cutting Edge Glaucoma Surgery: Will Viscocanalostomy Light the Way?, Supplement to the Review of Ophthalmology, Sept. 1999.

Welsh, N.H., FRCS et al., The "Deroofing" ofSchlemm's Canal in Patients with Open-Angle Glaucoma Through Placement of a Collagen Drainage Device, Ophthalmic Surgery and Lasers, March 1998, Vol. 29, No. 3, pp 216-226.

Tomihata, K., Ikada, Y., Cross-linking of hyaluronic acid with water-soluble carbodiimide, Journal Biomedical Material Research; 1997 John Wiley & sons, Inc, Vol 37; pgs 243-251.

T. Malson, P. Algvere, L. Ivert, B. Lindquist, G. Selen, S. Stenkula, Cross-linked hyaluronate gels for use in vitreous surgery, Biomaterials and Clinical Applications. Elsevier Science Publishers B. V. Amsterdam, 1987, pp 345-348.

E. Ghezzo, L. Benedetti, M. Rochirea, F. Biviano, L. Callegaro, Hyaluronan derivative microspheres as NGF delivery devices, preparation methods and in vitro release characterization, International Journal of Pharmacology, 87, pp 21-29, 1992.

### Object of the Invention:

It is the object of this invention to provide a biomaterial composition as defined in the claims for use in surgery, and ophthalmic surgery in particular. The biomaterial is comprised of an injectable microsphere formulation, wherein the microspheres are biocompatible, biodegradable and able to be delivered at high solids concentration. The material is capable of dilating tissues and forming an implant in-situ, while allowing for the passage of fluids through the resultant matrix of particles. Furthermore, it is an object of this invention to provide a formulation of microspheres which substantially reduces the tissue reaction in order to minimize the fibrotic healing response.

Due to the inherent biocompatibility of the stabilized microsphere compositions of the present invention, they are also applicable to the encapsulation or co-formulation of therapeutic and diagnostic compounds formulated for local or parenteral delivery.

### Summary of the Invention:

The present invention is directed at a novel microsphere composition comprising stabilized hyaluronic acid for use in direct contact with tissues for the purposes of surgery. In particular, the composition and use of such materials to manipulate tissues without the formation of a fibrotic response is described. Due to the inherent tissue biocompatibility of the microsphere formulations, there are additional uses for such materials in localized drug delivery and other medical applications.

There is provided herein a composition comprised of a biocompatible microsphere formulation which is flowable and biodegradable, the formulation is capable of being delivered to the operative site to effect the dilation or maintenance of a tissue space and allow for the flow of fluid through the microparticle matrix and to furthermore inhibit the deposition of fibrotic tissue. The formulation may be delivered by injection for surgical applications such as the dilation of Schlemm's Canal in the eye for the treatment of glaucoma, the angioplasty of small vessels, and as an aid in nerve reconstruction.

### Description of Invention:

This invention provides a flowable biomaterial as defined in the claims for use in surgery by administering the biomaterial in an amount sufficient to maintain a tissue space or deliver a sufficient amount of drug or active substance. In particular, the microsphere biomaterial composition is designed to be injected into Schlemm's Canal and other anatomic sites within the eye, producing tissue dilation and maintaining an increase in aqueous fluid outflow from the anterior chamber of the eye without causing a fibrotic response to close the tissue space.

The biomaterial of this invention is comprised of microparticles formed in a substantially spherical manner, or microspheres, suitably mixed into a physiologically compatible carrier solution. Due to the very small bores of needles needed for introduction into Schlemm's Canal, approximately 30 gauge or smaller, the flow characteristics of the biomaterial are important. In order to maximize injectability at high solid concentrations, dense microspheres are preferred to irregular shaped particles or fiber forms of microparticles. The microsphere are formed from hyaluronic acid. The microspheres are cross-linked to increase the biodegradation time in-situ. Microspheres of this invention will have diameters between 0.01 and 100 microns, preferably between 1 and 20 microns. The microspheres are suspended in a physiologic carrier solution such as phosphate buffered saline (PBS) or sterile water for injection (WFI). Microsphere concentrations in the formulation are in the range of 1% to 50% by weight, preferably greater than 2%.

The microspheres may be produced using standard spray drying techniques or may be produced by spray coagulation. Using spray drying techniques, an aqueous dispersion or colloid of the polymer is dispensed in atomized form through a small orifice nozzle into a flowing stream of gas, usually air or nitrogen. As the droplets fall in the gas stream, they condense and dry into substantially spherical particles of biomaterial. The particles are collected in a cyclone mechanism for further processing. In the technique of spray coagulation, a dispersion or colloidal solution of polymer is dispensed in atomized form through a small orifice nozzle into a receiver containing a solution which is a non-solvent of the polymer. Examples include isopropyl alcohol or ethyl alcohol. The droplets condense and dry through solvent exchange of the aqueous component. Appropriate condensation and solvent conditions are important for producing dense microspheres by this method.

The microspheres are stabilized to achieve non-solubility and to increase their degradation time in-vivo. The microspheres may be stabilized by a number of methods, including ionic complexation and chemical cross-linking. The microspheres may be cross-linked using a number of different chemistries, for example the use of a carbodiimide cross-linking agent. Agents to aid crosslinking may also be co-formulated into the microspheres. The hyaluronic acid starting material can be partially crosslinked to aid particle formation. After fabrication, methods of chemically cross-linking the microspheres in a non-hydrated or partially hydrated state act to increase the microsphere density. The cross-linked microspheres are washed to remove residual cross-linker and dried. The dry microspheres are then sized using standard sieving or filtration techniques to arrive at a population of the desired size range.

Microspheres are suspended in a physiologic carrier such as phosphate buffered saline, solutions of physiologically compatible surfactants, or dilute, buffered solutions of hyaluronic acid for delivery to the operative site. It can be readily appreciated that the microspheres may be size selected and stabilized to provide the appropriate residence time in-vivo, and formulated for a variety of medical applications. In practice for surgical use to treat Schlemm's Canal and other tissues in the eye, the space is located and accessed with a very fine gauge needle or cannula, with a subsequent injection of a slurry of the cross-linked microspheres. The semi-solid nature of the slurry provides sufficient dilating force to increase the approximate diameter of Schlemm's Canal. The high solids content of the slurry allows for the close packing of the microspheres, such that fluid can easily flow through the microsphere matrix and to the outflow channels of Schlemm's Canal.

In some cases, as in treating Schlemm's Canal of the eye, it may be beneficial to have a colored marker associated with the particles. The microspheres of the present invention or alternatively, the carrier fluid may be chemically treated to have an ionically bound or covalently bound chromophore or fluorophore. An example used in the bioconjugation field is fluorescein isothiocyanate, which would react with the reactive groups of hyaluronic acid to produce fluorescently tagged microspheres.

In other surgical applications where maintaining of space and anti-fibrotic properties are critically important, the formulations as described for treating Schlemm's Canal of the eye may be used. For example, the microsphere composition may be applied in the areas around nerves to reduce pressure induced complications or facilitate surgical repair, and similarly applied in the surgical treatment of reproductive, circulatory or digestive organs, situations where resulting fibrosis from wound healing would negate the effects of surgical repair. In another technique, dry microspheres, as described in the formulation, may be administered through aerosol spraying of the particles directly onto the moist surgical field. The microspheres will hydrate with serum and blood in the field.

The microsphere composition may be delivered by a variety of surgical instruments such injection needles, cannulas, and catheters. The flow properties of the composition may be adjusted for a particular application by control of microsphere size, swell, and concentration. Flow enhancing agents such as soluble hyaluronic acid, water soluble polymers, and surfactants may also be formulated into the composition.

Drugs or other active agents may be encapsulated, conjugated or co-formulated into the microsphere composition to provide local drug delivery. The drug may be chosen to aid the surgical application, such as by providing anti-inflammatory, anti-proliferative, or anti-fibrotic activity.

In addition to surgical applications, the microsphere compositions of the present invention provide an ideal carrier for therapeutic or diagnostic agents, due to their high degree of tissue and blood compatibility. Drugs for systemic treatment may be administered to a local site to provide predictable drug release characteristics due to the minimization of the fibrotic response. The microspheres also may be fabricated to allow parenteral administration by sizing the final particles to be smaller than a red blood cell, approximately 7 microns, to prevent trapping in capillaries. The microspheres are suspended in a physiologically compatible solution and injected into the blood circulation. Due to the blood compatibility of the HA surfaces exhibited by the microspheres produced, the microspheres resist removal from the circulatory system by the reticuloendothelial system of the liver and are capable of providing a sustained drug delivery effect.

### Examples:

### Example #1 - Fabrication of HA microspheres by spray coagulation

Microspheres comprised of hyaluronic acid (HA) were produced by spray formation and solvent drying. An aqueous solution of HA of 0.5% concentration is made up using highly purified HA and deionized water. The viscosity of the solution is lowered for spraying by the addition of isopropyl alcohol (IPA) in a ratio between 50:50 and 80:20 (IPA/aqueous), preferably in a ratio of 60% non-solvent.

The microspheres were formed by spraying the HA solution with a coaxial spray head wherein the inner bore carried the solution and the outer bore provided airflow for atomization. The inner bore was sized at 0.25 mm and the outer bore at 1.37 mm diameter. The spray head was arranged so as to spray downward into a collection vessel.

The collection vessel was filled approximately 5 cm deep with IPA as a non-solvent of the HA. Air at a pressure of 5 - 10 PSI was provided for atomization, and the solution was delivered via a standard syringe driven by either pneumatic or syringe pump drives. The air flow was activated prior to starting the HA solution flow. Microsphere diameters can be controlled by the diameter of the inner bore, air flow rate, solution viscosity and solution flow rate. By maintaining the inner bore, air flow rate and solution viscosities as constants, the solution flow rate was used to maintain size control.

As the solution exits the inner bore of the sprayer, it was atomized and the spherical droplets were carried by the air stream downward to enter the solvent bath in the collection vessel. The IPA non-solvent removed the remaining aqueous solution from the particles, thereby fixing them by coagulation. Particles formed in this manner were typically solid microspheres. The particles were essentially spherical with diameters ranging from 5-40 microns as determined by visual microscopy.

The coagulation bath solution was first filtered through a 45 micron mesh to remove any oversize microspheres. The solution was further filtered to collect desired size fractions. The final filtrate of the solution was then filtered through a 1.2 micron filter in order to collect the microspheres. The microspheres were washed from the collection filter and placed in a container. The microspheres were chemically cross-linked in a solution of 90% IPA and 10% aqueous solution of 10 mM of 1-ethyl-3-(3-dimethylaminopropyl) carbodiimide (EDC) for a period of 24 hours, washed with IPA, and dried by solvent drying.

### Example #2 - Fabrication of HA microspheres by ultrasonic spray coagulation

HA microspheres were produced using ultrasonic spray and solvent coagulation. A spray system consisting of a Lechler Model US-1 (Lechler AG, 100 kHz at 8 Watts maximum) ultrasound spray head directed to a collection vessel containing IPA as a non-solvent was employed. The ultrasound spray head was modified to decrease the bore diameter to 0.3 mm to produce smaller microspheres. The active portion of the spray head consisted of a titanium disc with a central bore for the delivery of the spray solution. An annular gap between the housing and the disc allowed for air flow to direct the spray and carry the particles in a desired direction. The spray head was powered by a RF amplifier system with variable power levels.

An aqueous solution of HA of 0.5% concentration was mixed with an equal amount of isopropyl alcohol (IPA) and allowed to mix thoroughly. IPA was added to lower the solution viscosity sufficiently for spraying. The solution was treated with EDC in an aqueous phase concentration of 50 milliMolar (mM) for a period of 24 hours. The EDC treatment of the HA solution formed cross-links and thereby increased the molecular weight of the HA and enhanced its film forming properties.

The microspheres were formed by dispensing the HA solution through the spray head using a syringe pump. The spray head was arranged so as to spray downward into a collection vessel. The collection vessel was filled approximately 5 cm deep with IPA as a non-solvent of the HA. Air at a pressure of 5 - 15 PSI was provided to help direct the spray downward. The ultrasound transducer and the air flow were activated prior to starting the HA solution flow. Flow rates of 0.1 to 2.0 cc/mm were used. The ultrasound power level was adjusted via the controller to provide the most consistent and smallest particle size.

As the atomized solution formed spherical droplets, they were carried by the air stream downward to enter the solvent bath in the collection vessel. The IPA non-solvent removed the remaining aqueous solution from the particles thereby fixing them by coagulation. Particles formed in this manner were typically thin-walled microspheres filled with liquid. The particles were essentially spherical with diameters ranging from 1-10 microns as determined by visual microscopy.

The coagulation bath solution was first filtered through a 20 micron mesh to remove any oversize microspheres. The final filtrate of the solution was then filtered through a 1.2 micron filter in order to collect the microspheres.

### Example #3 - Post Fabrication Cross-linking of Microspheres

Microspheres fabricated in the manner of either example 1 or 2 were collected and maintained in IPA. The microspheres were then cross-linked to stabilize them. A 100 mM solution of EDC was made up. The solution was added to the microspheres to achieve a final ratio of 90% IPA and 10% EDC solution. The microspheres were allowed to cross-link at 20° C for periods of 24 and 48 hours.

The cross-linked microspheres were then collected and washed three times with IPA to remove residual cross-linker. The resultant microspheres were placed on a glass slide and examined under a microscope. The microspheres maintained their shape and size during the processing. As the slide solution dried, a drop of water was placed on the slide and the particles examined. The particles showed very little change over a period of minutes. Another sample slide was prepared and a drop of 100 mM hydrochloric acid (HCL) was placed on the slide and the results observed. The microspheres showed evidence of hydration by the change in clarity of the wall, and diametrical swelling on the order of 10-30%. In contrast, microspheres which were not treated to the cross-linking process immediately swelled and began to dissolve in the acid solution, thereby indicating the success of the cross-linking process in producing high density microspheres.

### Example #4 - Injectable Formulation for HA Microspheres

Microspheres fabricated according to Examples #2 and #3 were produced. The microspheres were fractionated between 10 and 40 microns using successive filtration. The cross-linked microspheres were repeatedly washed with IPA to remove any residual aqueous component. The microspheres were collected by filtration through a 1.2 micron filter. The filter was dried in a low temperature oven at 150-175° C over a bed of desiccant.

Once dry, the microspheres were collected and weighed into a vial. DI water was added to the microspheres and mixed to result in a suspension of microspheres with a solids concentration of 2.6%. The solution was viscous but still able to be mixed at this high concentration. The suspension was dispensed through a micro-needle having an inner bore of 150 microns without difficulty.

### Example #5 - Injectable Formulation for HA Microparticles

Microspheres fabricated according to Examples #2 and #3 were produced. After cross-linking, the microspheres were concentrated by filter collection. The microspheres were size fractionated such that all particles were less than 4 microns in diameter in a hydrated state representative of physiological conditions.

The particles can be dried for storage. The concentrated IPA solution containing microspheres is cooled to -20° C and critical point dried. The remaining cake is comprised of hollow microspheres. The microspheres are resuspended in a solution of phosphate buffered saline to form an injectable formulation.

### Example #6 - Fluid Flow Through Microspheres

Microspheres were fabricated by spray coagulation of a solution of 1% HA, by the method described in Example #1. The microspheres were cross-linked with 50mM EDC using a solvent/aqueous ratio of 95:5 for a period of 118 hrs. The microspheres were washed, filtered to obtain the size fraction from 20-45 microns, then dried.

A 12.5% solids solution of the microspheres was formulated in deionized water. The microspheres were thoroughly mixed and allowed to fully hydrate. After hydration, an aliquot of microspheres was packed into the end of the Luer tube adapter of approximately 4 mm diameter to make a cake approximately 3 mm thick. A piece of nylon mesh filter with 10µ pores was cut and stretched over the end of the tube adapter, and held in place with a silicone O-ring around the outside to prevent extrusion of the particle matrix. The tube adapter was attached to a 60cc syringe barrel, which was held by a ring stand clamp in the vertical position. The syringe was filled to the 60cc mark with DI water, being careful to fill the tube adapter first so as not to trap an air bubble. The fluid was allowed to flow under the influence of gravity and atmospheric pressure only.

Within approximately 5 minutes, moisture was seen seeping through the nylon mesh. Within 60 minutes a full drop of water had accumulated on the mesh. Flow at this point remained small but continued steadily.

The experiment shows that a close packed matrix of cross-linked HA microspheres will allow fluid to flow with minimal pressure. The fluid transport in the interstitial spaces between particles, as well as through the hydrated particles sets up a steady flow of fluid through the matrix.

### Example #7 - Surgical Use of HA Microspheres

A microsphere formulation according to Example 4 was produced and loaded into a 1 ml syringe. Using a 20 gauge needle, the material was injected into an ex-vivo sample of muscle tissue, causing local tissue dilation and expansion around the injection site. Examination of the injection site by dissection and microscopy demonstrated a collection of microspheres forming a coherent mass implant at the injection site.

### Example #8 - Drug Delivery Reservoir Use of HA Microspheres

A microsphere formulation according to Example 4 is produced and placed into a 1 ml syringe. With a syringe needle, the material is injected into the soft connective tissue of a mammal to create an implant mass capable of slow release of drug incorporated into the microsphere formulation.

### Example #9 - Parenteral Use of HA. Microspheres

A microsphere formulation according to Example 5 was produced with a resulting microsphere concentration of approximately 1 wt%. The injectable formulation is injected intravenously into a test animal, resulting in a time dependent concentration of circulating microspheres in the blood stream without adverse physiological effect.

## Claims

1. An injectable composition for use in direct tissue contact, comprising microspheres of stabilized hyaluronic acid, wherein said microspheres are substantially spherical and uniform such that close packing rules apply to a mass of said microspheres providing an implant with interconnected porosity when injected into tissues, wherein said interconnected porosity allows for the free transport of fluid in-vivo, and the resultant implant demonstrates antifibrotic properties.

2. A composition according to claim 1, wherein said microspheres have at least one hollow core.

3. A composition according to claim 1, wherein said microspheres are produced by spray drying.

4. A composition according to claim 1, wherein said microspheres are produced by coagulation of a solution of a biomaterial introduced into a non-solvent of said biomaterial.

5. A composition according to claim 1, wherein said microspheres are ionically cross-linked.

6. A composition according to claim 1, wherein said microspheres are chemically cross-linked.

7. A composition according to claim 1, wherein said microspheres are chemically cross-linked in a solvent mixture comprising an organic solvent.

8. A composition according to claim 7, wherein the cross-linked microspheres exhibit a fluid uptake of between 10% and 1,000% by weight.

9. A composition according to claim 1, wherein said microspheres further comprise crosslinking agents.

10. A composition according to claim 1, wherein said microspheres are chemically cross-linked using a water-soluble carbodiimide cross-linking agent.

11. A composition according to claim 1, wherein said microspheres are between 0.5 and 100 microns in average diameter.

12. A composition according to claim 1, wherein said microspheres are between 0.5 and 20 microns in average diameter.

13. A composition according to claim 1, wherein the composition additionally comprises protease inhibitors, anti-proliferative agents, anti-fibrosis or anti-inflammatory agents.

14. A composition according to claim 1, wherein said microspheres also contain a therapeutic or diagnostic agent.

15. A composition according to claim 1, wherein said microspheres are suspended in a fluid medium at a concentration of between 2% and 50 % by weight.

16. A composition according to claim 15, wherein said fluid medium comprises water.

17. A composition according to claim 15, wherein said fluid medium is buffered to a near physiologic pH.

18. A composition according to claim 15, wherein said fluid medium comprises a buffered dispersion of non-cross-linked hyaluronic acid.

19. A composition according to claim 15, wherein said microspheres allow dilation of a tissue space when injected through a syringe needle or cannula.

20. A composition according to claim 1, wherein said composition is injectable through a syringe needle or cannula.

21. A composition according to claim 1, wherein said composition is injectable through a 30 gauge needle or cannula.

22. A composition according to claim 1, wherein said composition additionally comprises a colored or fluorescent dye.

23. A kit for for surgical manipulation of tissues by localized injection of microspheres comprising:
- the injectable composition of any of claims 1 to 22;
- a means to inject said composition into a local area of tissue.

24. A kit according to claim 23, wherein the means to inject said composition comprises a needle of bore size 30 gauge or smaller.

25. A kit according to claim 23 or daim 24, wherein the means to inject said composition is a microcannula configured for injection of said composition into Schlemm's Canal of the eye.

26. A kit according to claim 25, wherein a colored marker is associated with the injectable composition of microspheres.

## Patentansprüche

1. Injizierbare Zusammensetzung zur Anwendung in direktem Gewebekontakt, umfassend Mikrokugeln aus stabilisierter Hyaluronsäure, wobei die Mikrokugeln im wesentlichen kugelförmig und gleichförmig sind, so dass Regeln der dichten Packung für eine Masse dieser Mikrokugeln gelten, wobei bei Injektion in Gewebe ein Implantat mit verbundener Porosität bereitgestellt wird, wobei diese verbundene Porosität den freien Transport von Fluid in vivo ermöglicht, und das resultierende Implantat antifibrotische Eigenschaften zeigt.

2. Zusammensetzung gemäß Anspruch 1, worin die Mikrokugeln mindestens einen hohlen Kern aufweisen.

3. Zusammensetzung gemäß Anspruch 1, worin die Mikrokugeln mittels Sprühtrocknung hergestellt werden.

4. Zusammensetzung gemäß Anspruch 1, worin die Mikrokugeln mittels Koagulation einer Lösung eines Biomaterials, das in ein Nicht-Lösungsmittel dieses Biomaterials eingeführt wurde, hergestellt werden.

5. Zusammensetzung gemäß Anspruch 1, wobei die Mikrokugeln ionisch vernetzt werden.

6. Zusammensetzung gemäß Anspruch 1, wobei die Mikrokugeln chemisch vernetzt werden.

7. Zusammensetzung gemäß Anspruch 1, worin die Mikrokugeln in einer Lösungsmittelmischung, die ein organisches Lösungsmittel umfasst, chemisch vernetzt werden.

8. Zusammensetzung gemäß Anspruch 7, worin die vernetzten Mikrokugeln eine Fluidaufnahme zwischen 10 Gew.-% und 1000 Gew.-% aufweisen.

9. Zusammensetzung gemäß Anspruch 1, wobei die Mikrokugeln zusätzlich Vernetzungsmittel umfassen.

10. Zusammensetzung gemäß Anspruch 1, wobei die Mikrokugeln unter Verwendung eines wasserlöslichen Carbodümid-Vernetrungsmittels chemisch vernetzt werden.

11. Zusammensetzung gemäß Anspruch 1, wobei die Mikrokugeln einen durchschnittlichen Durchmesser im Bereich von 0,5 bis 100 µm aufweisen.

12. Zusammensetzung gemäß Anspruch 1, wobei die Mikrokugeln einen durchschnittlichen Durchmesser im Bereich von 0,5 bis 20 µm aufweisen.

13. Zusammensetzung gemäß Anspruch 1, wobei die Zusammensetzung zusätzlich Protease-Inhibitoren, antiproliferative Mittel, antifibrotische oder antiinflammatorische Mittel umfasst.

14. Zusammensetzung gemäß Anspruch 1, worin die Mikrokugeln ferner ein therapeutisches oder diagnostisches Mittel enthalten.

15. Zusammensetzung gemäß Anspruch 1, worin die Mikrokugeln in einem Fluidmedium in einer Konzentration zwischen 2 Gew.-% und 50 Gew.-% suspendiert werden.

16. Zusammensetzung gemäß Anspruch 15, wobei das Fluidmedium Wasser umfasst.

17. Zusammensetzung gemäß Anspruch 15, wobei das Fluidmedium auf einen ungefähr physiologischen pH gepuffert ist.

18. Zusammensetzung gemäß Anspruch 15, wobei das Fluidmedium eine gepufferte Dispersion von nichtvernetzter Hyaluronsäure umfasst.

19. Zusammensetzung gemäß Anspruch 15, worin die Mikrokugeln eine Dilatation eines Geweberaums ermöglichen, wenn sie mittels einer Spritzennadel oder Kanüle injiziert werden.

20. Zusammensetzung gemäß Anspruch 1, worin die Zusammensetzung durch eine Spritzennadel oder Kanüle injizierbar ist.

21. Zusammensetzung gemäß Anspruch 1, worin die Zusammensetzung durch eine 30-Gauge-Nadel oder -Kanüle injizierbar ist.

22. Zusammensetzung gemäß Anspruch 1, worin die Zusammensetzung zusätzlich einen farbigen oder fluoreszierenden Farbstoff enthält.

23. Kit zur chirurgischen Manipulation von Geweben durch lokalisierte Injektion von Mikrokugeln, umfassend
- die injizierbare Zusammensetzung gemäß einem der Ansprüche 1 bis 22,
- Mittel zum Injizieren dieser Zusammensetzung in einen lokalen Bereich eines Gewebes.

24. Kit gemäß Anspruch 23, worin das Mittel zum Injizieren der Zusammensetzung eine Nadel mit einem 30-Gauge-Durchmesser oder weniger umfasst.

25. Kit gemäß Anspruch 23 oder 24, worin es sich bei dem Mittel zum Injizieren der Zusammensetzung um eine Mikrokanüle handelt, die zur Injektion dieser Zusammensetzung in den Schlemm-Kanal des Auges konfiguriert ist.

26. Kit gemäß Anspruch 25, worin ein farbiger Marker mit der injizierbaren Zusammensetzung aus Mikrokugeln assoziiert ist.

## Revendications

1. Composition injectable pour l'utilisation dans un contact tissulaire direct, comprenant des microsphères d'acide hyaluronique stabilisé, dans laquelle lesdites microsphères sont sensiblement sphériques et uniformes de sorte que les règles de remplissage des doses s'appliquent à une masse desdites microsphères fournissant un implant avec une porosité interconnectée lors de l'injection dans des tissus, dans laquelle ladite porosité interconnectée permet le transport libre d'un liquide in vivo, et l'implant résultant montre des propriétés anti-fibreuses.

2. Composition selon la revendication 1, dans laquelle lesdites microsphères ont au moins un centre creux.

3. Composition selon la revendication 1, dans laquelle lesdites microsphères sont produites par séchage par atomisation.

4. Composition selon la revendication 1, dans laquelle lesdites microsphères sont produites par coagulation d'une solution d'un biomatériau introduit dans un non-solvant dudit biomatériau.

5. Composition selon la revendication 1, dans laquelle lesdites microsphères sont ioniquement réticulées.

6. Composition selon la revendication 1, dans laquelle lesdites microsphères sont chimiquement réticulées.

7. Composition selon la revendication 1, dans laquelle lesdites microsphères sont chimiquement réticulées dans un mélange de solvant comprenant un solvant organique.

8. Composition selon la revendication 7, dans laquelle les microsphères réticulées présentent une capture du liquide d'entre 10 % et 1 000 % en poids.

9. Composition selon la revendication 1, dans laquelle lesdites microsphères comprennent en outre des agents de réticulation.

10. Composition selon la revendication 1, dans laquelle lesdites microsphères sont chimiquement réticulées en utilisant un agent de réticulation de carbodiimide hydrosoluble.

11. Composition selon la revendication 1, dans laquelle lesdites microsphères ont un diamètre moyen d'entre 0,5 et 100 microns.

12. Composition selon la revendication 1, dans laquelle lesdites microsphères ont un diamètre moyen d'entre 0,5 et 20 microns.

13. Composition selon la revendication 1, dans laquelle la composition comprend de manière supplémentaire des inhibiteurs de protéase, des agents anti-proliférants, des agents anti-fibreux ou anti-inflammatoires.

14. Composition selon la revendication 1, dans laquelle lesdites microsphères contiennent également un agent thérapeutique ou diagnostique.

15. Composition selon la revendication 1, dans laquelle lesdites microsphères sont en suspension dans un milieu liquide à une concentration comprise entre 2 % et 50 % en poids.

16. Composition selon la revendication 15, dans laquelle ledit milieu liquide comprend de l'eau.

17. Composition selon la revendication 15, dans laquelle ledit milieu liquide est tamponné à un pH proche du pH physiologique.

18. Composition selon la revendication 15, dans laquelle le milieu liquide comprend une dispersion tamponnée d'acide hyaluronique non réticulé.

19. Composition selon la revendication 15, dans laquelle lesdites microsphères permettent la dilatation d'une espace tissulaire lors de l'injection par une aiguille de seringue ou une canule.

20. Composition selon la revendication 1, dans laquelle ladite composition est injectable par une aiguille de seringue ou une canule.

21. Composition selon la revendication 1, dans laquelle ladite composition est injectable par une aiguille de grosseur 30 ou une canule.

22. Composition selon la revendication 1, dans laquelle ladite composition comprend en plus un colorant coloré ou fluorescent.

23. Kit pour la manipulation chirurgicale des tissus par une injection localisée de microsphères comprenant :
- la composition injectable selon l'une quelconque des revendications 1 à 22 ;
- un moyen pour injecter ladite composition dans une zone locale de tissu.

24. Kit selon la revendication 23, dans lequel le moyen pour injecter ladite composition comprend une aiguille de calibre 30 ou moins.

25. Kit selon la revendication 23 ou la revendication 24, dans lequel le moyen pour injecter ladite composition est une microcanule configurée pour l'injection de ladite composition dans le canal de Schlemm de l'oeil.

26. Kit selon la revendication 25, dans lequel un marqueur coloré est associé à la composition injectable de microsphères.
